# EUROPEAN PATENT SPECIFICATION

(11) **EP 1 237 860 B1**
(45) Date of publication and mention of the grant of the patent: **25.02.2004**
(21) Application number: 00985524.8
(22) Date of filing: 11.12.2000
(51) Int. Cl.: C07C 327/42, C22B 3/34, G01N 27/333

(54) **CALIXARENES**
CALIXARENE
CALIXARENES

(30) Priority: 13.12.1999 US 460237; 03.05.2000 GB 0010551
(43) Date of publication of application: 11.09.2002
(73) Proprietor: THE SECRETARY OF STATE FOR DEFENCE, London SW1A 2HB (GB)
(72) Inventor: NICHOLSON, Graeme Peter, Chemical Analysis Branch, Reading RG7 4PR (GB); KAN, Mark Joseph, Chemical Analysis Branch, Reading RG7 4PR (GB); WILLIAMS, Gareth, Chemical Analysis Branch, Reading RG7 4PR (GB); DREW, Michael George, Chemistry Dept., Reading RG6 2AD (GB); BEER, Paul Derek, Inorganic Chemistry Laboratory, Oxford OX1 3QR (GB)
(74) Representative: Skelton, Stephen Richard
(86) International application number: PCT/GB2000/004729
(87) International publication number: WO 2001/044175

(56) References cited:
- EP-A- 0 432 989
- WO-A-97/17322
- P.L.H.M. COBBEN ET AL: J. AM. CHEM. SOC., vol. 114, no. 26, 1992, pages 10573-10582, XP002162182

## Description

This invention relates to novel calixarenes, methods of their preparation and uses thereof, in particular for the sequestration of metals.

Calixarenes have been suggested as being useful for the sequestration of certain ionic metallic species. European Patent Publication No. 0 432 989 and WO 97/17322 describe a number of calixarene and oxacalixarene derivatives having metal sequestering properties and review some of the prior art in this field. It is the object of the present invention to provide calixarenes with an improved ability to sequester metals.

In accordance with the present invention, calixarenes of a formula (I) 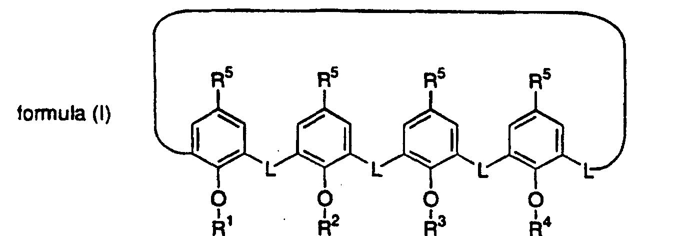 wherein:
L is [ - CH₂ - ] or [ -O-CH₂-O- ] and is the same or different between each aryl group;
R⁵ is H, -NO₂, halogen, or C₁ - C₁₀ aliphatic hydrocarbyl group, C₆ - C₂₀ aryl group, C₆ - C₂₀ hydrocarbylaryl group, any of which is optionally substituted by one or more halo or oxo groups or interrupted by one or more oxo groups, and R⁵ is the same or different on each aryl group;
R¹ comprises a carboxy group which is or is not protonated or protected;
two groups out of R², R³ and R⁴ are H; and
the one group out of R², R³ and R⁴ not being H comprises a thioamide group.

The combination of acid/ester and thioamide groups is not found in the prior art and leads to improved metal sequestration properties.

Most preferably, R² and R⁴ are H and R³ comprises a thioamide group.

L is preferably [-CH₂-] between each of the aryl groups and R⁵ may be a tertiary butyl.

In a preferred embodiment, the carboxy group R¹ conforms to the general formula (A):

(A) [-X-COOR¹⁰]

wherein X is a C₁, a C₂ or a C₃ carbon chain which is a part of an aliphatic hydrocarbyl group, aryl group or hydrocarbylaryl group, any of which is optionally substituted by one or more halo, oxo or nitro groups; and R¹⁰ is H or a protecting group being a salt or an ester group.

Furthermore, in a preferred embodiment R¹⁰ is H and the aliphatic hydrocarbyl group, aryl group or hydrocarbylaryl group of formula (A) is substituted by one or more groups which cause a reduction in the pKa of the carboxylic acid group with respect to an unsubstituted molecule.

Most preferably, R¹ is of the general formula (B):

(B) [ - (C.R⁶.R⁷)ₙ-COOR¹⁰]

wherein n is 1, 2 or 3 and R⁶ and R⁷ are H or halogen and is the same or different on each carbon.

Alternatively, R¹ is of the general formula (C): 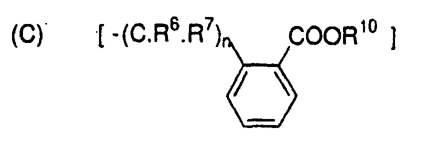 wherein n is 0 or 1 and R⁶ and R⁷ are H or halogen and is the same or different on each carbon and wherein the phenyl ring of the benzoic acid group is optionally substituted by one or more halo, oxo or nitro groups. In this embodiment, R¹⁰ may be H and the phenyl ring of the benzoic acid of formula (C) may be substituted by one or more groups which cause a reduction in the pKa of the carboxy group with respect to an unsubstituted molecule.

In structures (B) and (C) above, n is preferably 1 and R⁶ and R⁷ are preferably both H.

In unprotected acid embodiments, the aliphatic hydrocarbyl group, aryl group or hydrocarbylaryl group of X in formula (A) is preferably substituted by one or more groups causing a reduction in the pKa of the carboxy group with respect to the unsubstituted molecule.

It is preferred that the thioamide group R², R³ or R⁴ of formula (I) is of the general formula (D): 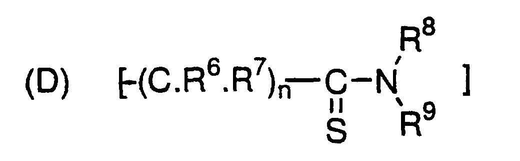 wherein n is 1, 2 or 3 and R⁶ and R⁷ are H, halogen, or C₁-C₁₀ aliphatic hydrocarbyl group, and is the same or different on each carbon, and wherein R⁸ and R⁹, which is the same or different, are H or C₁ - C₁₀ aliphatic hydrocarbyl group which is optionally substituted by one or more halo groups, or is optionally interrupted by a keto group or is optionally a cycloaliphatic ring formed by R⁸ and R⁹ together, or is optionally conjugated to a second calixarene.

In a further preferred embodiment the calixarene is of formula (II)

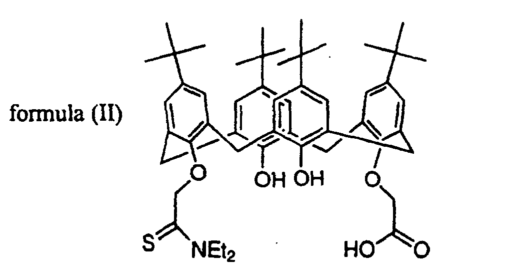

It is optional that where calixarenes are of the formulae (I) or (II) then some or all of phenyl groups of the calixarene ring are further peripherally substituted, in such a way that the sequestering abilities of the calixarene are not compromised.

In a further embodiment of the invention, a dimer can be made wherein one of the R⁸ and R⁹ groups is conjugated to a second calixarene.

The R⁸ or R⁹ group of one calixarene can be conjugated to the R⁸ or R⁹ group of the other calixarene, optionally through a spacer group R¹¹, the optional spacer group R¹¹ being C₁-C₆ aliphatic hydrocarbyl group, C₆-C₁₀ aryl group, C₆-C₁₆ hydrocarbylaryl group, any of which may optionally be substituted by one or more halo or oxo groups or interrupted by one or more oxo groups.

In a second aspect of the invention, there is provided a method of sequestering metals comprising contacting the metals with a calixarene in accordance with the present invention.

Preferably, the method is carried out at a pH of between 2 and 11. It is most preferable that the pH at which the method is carried is buffered. The buffer used may be citrate. A low pH will decrease the likelihood of metal precipitation, whereas a high pH will increase the dissociation of unprotected acid groups.

The method may comprise the following steps:
(i) dissolving the calixarene in an hydrophobic organic solvent;
(ii) mixing the organic solvent with an aqueous phase containing metal ions;
(iii) agitating the organic solvent and aqueous phase together;
(iv) recovering the metal from the organic phase.

This method enables the recovery of the extracted metal from the organic phase by contacting with a relatively small volume of acid.

The method may conveniently be used to sequester a metal chosen from a Lanthanide, U, Hg, Am, Pb, Sr, Bi, Cd, Ag and Y. The calixarene should be present in excess.

In the method, the calixarene may optionally be solid phase bound e.g. in a polymer support. This should be readily achieved by the person skilled in the art, given the disclosures concerning the use of polymer supports in US 4 642 362, 4 699 966 and 4 447 585 and in European Patent publication no. 0 217 656.

In a third aspect of the invention, the calixarenes can be incorporated into an ion-selective polymeric membrane for an electrochemical sensor comprising a supporting matrix.

In a fourth aspect of the invention, a process for preparing the calixarenes in accordance with the present invention comprises the sequential steps of:
(i) bis-esterification of a calix[4]arene;
(ii) deprotection of a first ester group to form a first acid group;
(iii) chlorination of the first acid group to form an acyl chloride;
(iv) substitution of the chlorine group in the said acyl chloride with a diamine moiety to form an amide group; and
(v) substitution of the oxygen group in the amide group with a sulphur group toform a thioamide moiety.

This produces ester-thioamides in good yield and is a relatively simple process.

The process may further comprise a subsequent step of deprotecting the second ester group to form a second acid group. This produces an acid-thioamide in good yield and is relatively simple.

The invention will now be described by way of example only with reference to the following drawings of which:

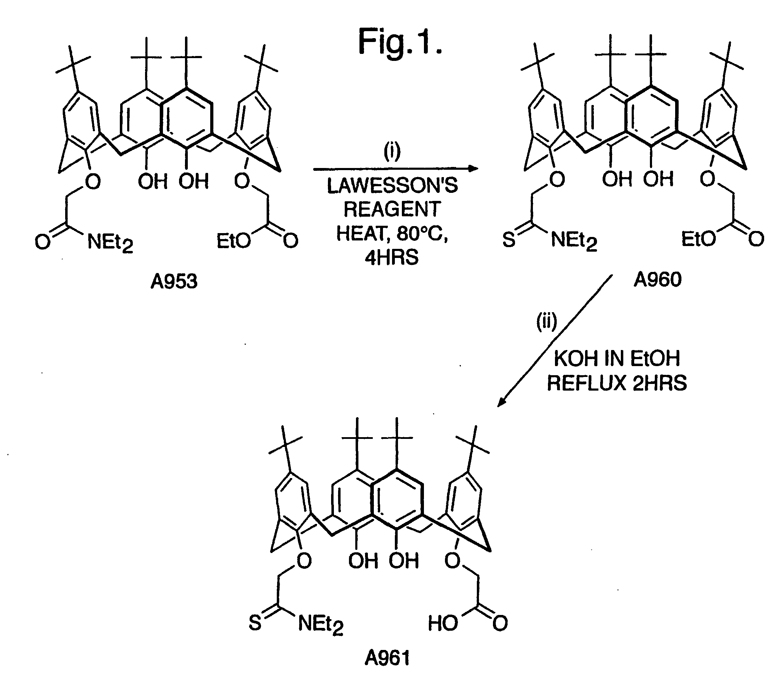

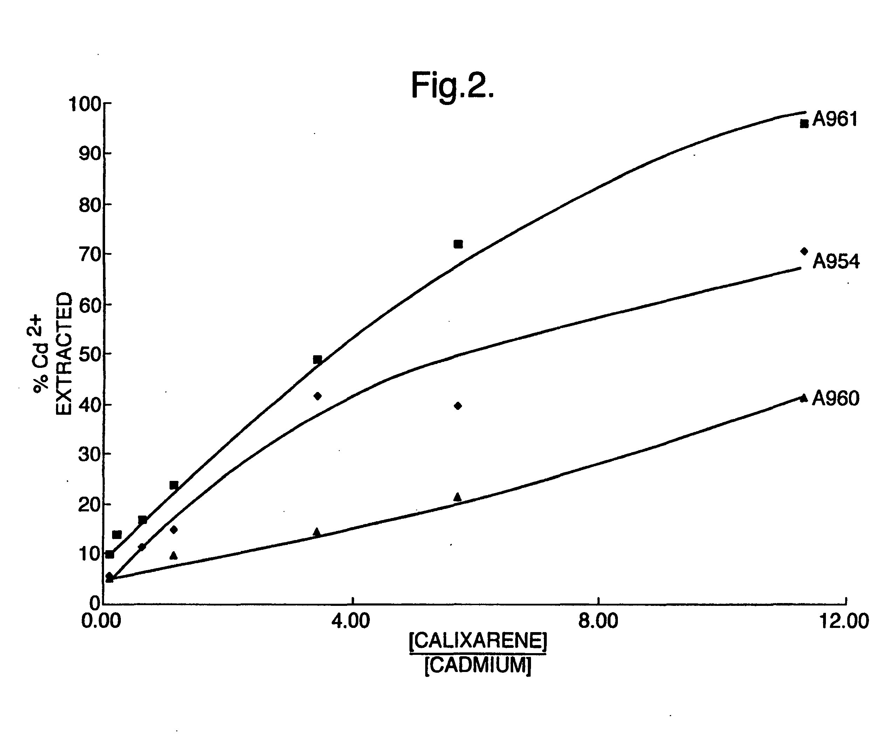

Figure 1 is a schematic showing a synthetic route for the ester-thioamide A960 and acid-thioamide A961, both compounds being in accordance with the present invention; and Figure 2 shows the variation in % extraction of Cadmium (Cd²⁺) ions with the molar ratio of Calixarene:Cd²⁺ for prior art compound acid-amide A954 (patent application number WO 97/17322, Fig. 15), and two materials in accordance with the present invention (ester-thioamide A960 and acid-thioamide A961) at pH=9.4.

### Example 1 - synthesis of the ester-thioamide A960

A960 was synthesised using the route shown in Figure 1. The precursor A953 was prepared in accordance with patent publication number WO 97/17322. Lawesson's reagent (0.49 g, 1.2 mmol) was added to a solution of ester-amide A953 (1.0g, 1.17 mmol) in toluene (20 cm³) and the mixture was heated at 80 °C for 4 hr. After cooling to room temperature, the toluene was removed under reduced pressure to give a yellow oil. This oil was dissolved in acetonitrile (15 cm³) and filtered through an alumina pad. Dropwise addition of water to the filtrate afforded a yellow precipitate, which was removed by filtration and recrystallised from dichloromethane-ethanol to afford A960 as yellow prismatic crystals (0.95g, 94%). The structure of this compound was confirmed by nuclear magnetic resonance spectroscopy (NMR), mass spectrometry and X-ray crystallography.

### Example 2 - synthesis of the acid-thioamide A961

A961 was synthesised using the route shown in Figure 1. The ester-thioamide A960 was synthesised by the route shown in Figure 1 and Example 1. Potassium hydroxide (0.036g, 0.65 mmol) was added to a solution of ester-thioamide A960 (0.5g, 0.58 mmol) in ethanol (25 cm³) and the solution heated under reflux for 2 hr. The ethanol was reduced in volume to approximately 5 cm³ and 1M HCl added to precipitate A961 as a pale yellow powder which was recrystallised from dichloromethane-hexane (0.41g, 85%). The structure of this compound was confirmed by NMR and mass spectrometry.

The synthesis of other related compounds in accordance with the present invention can be readily undertaken by one skilled in the art using the teaching of the present invention in association with the teaching of patent publication number WO97/17322.

### Example 3 - ability of the compounds in accordance with the present invention to sequester metals

Figure 2 shows the ability of the calixarenes in accordance with the present invention A960 and A961 to extract cadmium ions at pH 9.4. Data are also presented for a prior art material, A954 (prepared in accordance with patent application, publication number WO97/17322, example 11 and figure 15 therein). Equal volumes of aqueous cadmium cyanide solution (pH 9.4, [Cd²⁺]=0.238mMofar) and a solution of a calixarene in dichloromethane were mixed for 15 minutes by stirring. The aqueous and organic phases were then allowed to separate for about 30 minutes. The aqueous layer (Aq1) was then removed, and the organic layer was washed with a nitric acid blank (pH 9.4). The aqueous and organic layers were allowed to separate for about 30 minutes, and the aqueous layer was then removed (Aq2). Aq1 contained the cadmium ions that had not been extracted by the calixarenes, whereas Aq2 contained the cadmium ions that had been extracted by the calixarenes (and subsequently liberated by acidification of the organic layer). Aq1 and Aq2 were made up to known volumes. ICP AES (inductively coupled plasma atomic emission spectroscopy) was then used to determine the concentration of cadmium ions in the solutions. These figures can readily be used to determine the percentage extraction of cadmium for a given ratio of concentration of calixarene:cadmium.

Figure 2 indicates that both the acid-thioamide A961 and the ester-thioamide A960 are capable of extracting cadmium ions from solution. The order of efficiency of extraction is acid-thioamide, A961 > acid-amide, A954 > ester-thioamide, A960. The order can be explained by the fact that both A961 and A954 have a proton that can be readily lost from the acid substituent. The resulting anion will attract and retain cadmium ions more effectively than the (usually uncharged) ester group. The acid-thioamide (A961) forms complexes with cadmium more readily than the acid-amide (A954) because the S atom in A961 is a "softer" atom than the O atom in A954, and is thus more polarisable and thus is more likely to form a complex with a Cd²⁺ ion, which is itself a "soft" ion.

Hence, the compounds in accordance with the present invention may be used in applications in which the sequestration of metal ions is important, such as liquid waste decontamination and water purification.

Furthermore, the compounds in accordance with the present invention may be used in sensors, wherein the compounds form part of an ion-selective polymeric membrane for an electrochemical sensor. The ionophore is dispersed within a supporting matrix. The matrix is polymeric and also typically comprises an ion exchange material such as potassium tetra parachlorophenyl borate. The supporting membrane may further comprise a plasticiser such as 2-nitrophenyl octyl ether, dioctylphthalate, dibutylsebacate and dioctylphenylphosphonate. The membrane will be attached to an electrode and the presence of selective ions will cause a change in the electrical characteristics of the electrode (relative to a reference electrode, such as a Calomel electrode) and thus be registered by the sensor.

## Claims

1. Calixarenes of a formula (I) 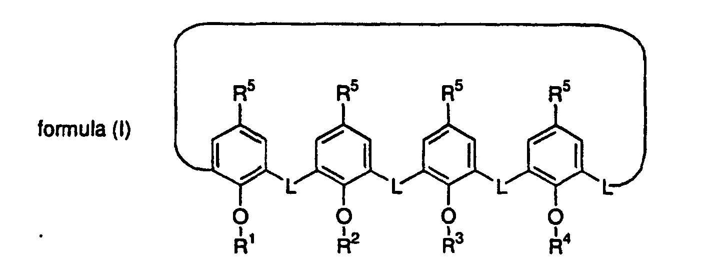 wherein:
L is [ - CH₂ - ] or [ -O-CH₂-O- ] and is the same or different between each aryl group;
R⁵ is H, NO₂, halogen, or C₁ - C₁₀ aliphatic hydrocarbyl group, C₆ - C₂₀ aryl group, C₆ - C₂₀ hydrocarbylaryl group, any of which is optionally substituted by one or more halo or oxo groups or interrupted by one or more oxo groups, and R⁵ is the same or different on each aryl group;
R¹ comprises a carboxy group which is or is not protonated or protected;
two groups out of R², R³ and R⁴ are H; and
the one group out of R², R³ and R⁴ not being H comprises a thioamide group.

2. Calixarenes as claimed in claim 1 wherein R² and R⁴ are H and R³ comprises a thioamide group.

3. Calixarenes as claimed in claim 1 or claim 2 wherein L is [- CH₂ - ] between each of the aryl groups.

4. Calixarenes as claimed in any one of claims 1 to 3 wherein R⁵ is a tertiary butyl.

5. Calixarenes as claimed in any one of claims 1 to 4 wherein the carboxy group R' conforms to the general formula (A):
(A) [ - X - COOR¹⁰]
wherein X is a C₁, a C₂ or a C₃ carbon chain which is a part of an aliphatic hydrocarbyl group, aryl group or hydrocarbylaryl group, any of which is optionally substituted by one or more halo, oxo or nitro groups; and R¹⁰ is H or a protecting group being a salt or an ester group.

6. Calixarenes as claimed in claim 5 wherein R¹⁰ is H and the aliphatic hydrocarbyl group, aryl group or hydrocarbylaryl group of formula (A) are substituted by one or more groups which cause a reduction in the pKa of the carboxylic acid group with respect to an unsubstituted molecule.

7. Calixarenes as claimed in claim 5 wherein R' is of the general formula (B):
(B) [-(C.R⁶.R⁷)ₙ-COOR¹⁰]
wherein n is 1, 2 or 3 and R⁶ and R⁷ are H or halogen and is the same or different on each carbon.

8. Calixarenes as claimed in claim 5 wherein R¹ is of the general formula (C): 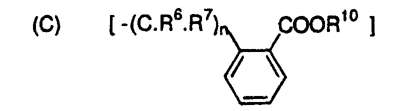 wherein n is 0 or 1 and R⁶ and R⁷ are H or halogen and is the same or different on each carbon and wherein the phenyl ring of the benzoic acid group is optionally substituted by one or more halo, oxo or nitro groups.

9. Calixarenes as claimed in claim 8 wherein R¹⁰ is H and the phenyl ring of the benzoic acid of formula (C) is substituted by one or more groups which cause a reduction in the pKa of the carboxy group with respect to an unsubstituted molecule.

10. Calixarenes as claimed in any one of claims 5 to 9 wherein n is 1 and R⁶ and R⁷ are both H.

11. Calixarenes as claimed in any one of the preceding claims wherein the thioamide group R², R³ or R⁴ of formula (I) is of the general formula (D): 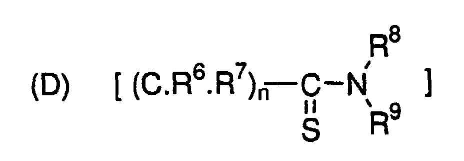 wherein n is 1, 2 or 3 and R⁶ and R⁷ are H, halogen, or C₁-C₁₀ aliphatic hydrocarbyl group, and is the same or different on each carbon, and wherein R⁸ and R⁹, which is the same or different, are H or C₁ - C₁₀ aliphatic hydrocarbyl group which is optionally substituted by one or more halo groups, or is optionally interrupted by a keto group or is optionally a cycloaliphatic ring formed by R⁸ and R⁹ together, or is optionally conjugated to a second calixarene.

12. A calixarene of formula (II)

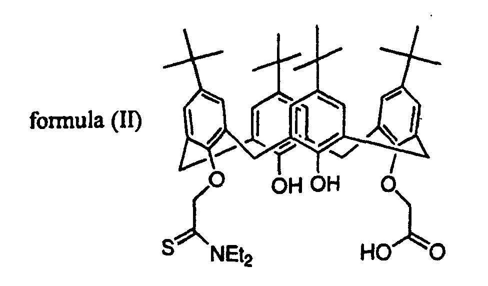

13. Calixarenes of the formulae (I) or (II) as claimed in claim 1 or 12 wherein some or all of phenyl groups of the calixarene ring are further peripherally substituted.

14. A calixarene dimer comprising a calixarene as claimed in either claim 11 wherein one of the R⁸ and R⁹ groups is conjugated to a second calixarene.

15. A dimer as claimed in claim 14 wherein the R⁸ or R⁹ group of one calixarene is conjugated to the R⁸ or R⁹ group of the other calixarene, optionally through a spacer group R¹¹, the optional spacer group R¹¹ being C₁-C₆ aliphatic hydrocarbyl group, C₆-C₁₀ aryl group, C₆-C₁₆ hydrocarbylaryl group, any of which may optionally be substituted by one or more halo or oxo groups or interrupted by one or more oxo groups.

16. A method of sequestering metals comprising contacting the metals with a calixarene as claimed in any one of the preceding claims.

17. The method as claimed in claim 16 wherein the method is carried out at a pH of between 2 and 11.

18. The method as claimed in claim 16 or 17 wherein the pH at which the method is carried is buffered.

19. The method as claimed in claim 18 wherein the buffer used is citrate.

20. The method as claimed in any one of claims 16 to 19 comprising the following steps:
(i) dissolving the calixarene in an hydrophobic organic solvent;
(ii) mixing the organic solvent with an aqueous phase containing metal ions;
(iii) agitating the organic solvent and aqueous phase together;
(iv) recovering the metal from the organic phase.

21. The method as claimed in any one of claims 16 to 20 wherein the metal is selected from a Lanthanide, U, Hg, Am, Pb, Sr, Bi, Cd, Ag and Y.

22. The method as claimed in any one of claims 16 to 21 further **characterised in that** the calixarene is solid phase bound.

23. An ion-selective polymeric membrane for an electrochemical sensor comprising a supporting polymer matrix and a calixarene as claimed in any one of claims 1 to 15.

24. A process for preparing a calixarene as claimed in any one of claims 1 to 15 comprising the sequential steps of:
(i) bis-esterification of a calix[4]arene;
(ii) deprotection of a first ester group to form a first acid group;
(iii) chlorination of the first acid group to form an acyl chloride;
(iv) substitution of the chlorine group in the said acyl chloride with a diamine moiety to form an amide group; and
(v) substitution of the oxygen group in the amide group with a sulphur group to form a thioamide moiety.

25. A process as claimed in claim 24, wherein the process comprises a subsequent step of deprotecting the second ester group to form a second acid group.

## Patentansprüche

1. Calixarene mit der Formel (I) 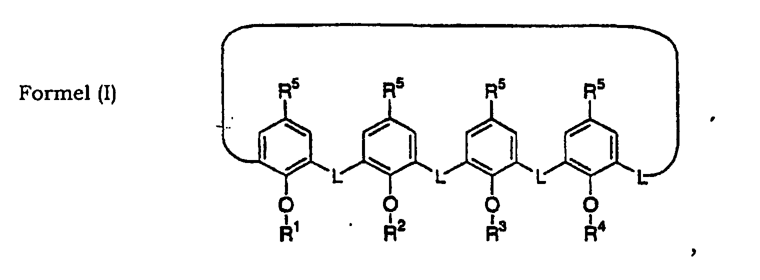 worin:
- L [-CH₂-] oder [-O-CH₂-O-] bedeuten und zwischen den jeweiligen Arylgruppen gleich oder voneinander verschieden sind,
- R⁵ H, -NO₂, ein Halogenatom oder eine aliphatische C₁- bis C₁₀-Kohlenwasserstoffgruppe, C₆-C₂₀-Arylgruppe, C₆- bis C₂₀-Kohlenwasserstoffarylgruppe bedeutet, wobei jede davon wahlweise durch eine oder mehrere Halogen- oder Oxo-Gruppen substituiert oder durch eine oder mehrere Oxogruppen unterbrochen ist und die R⁵ an den jeweiligen Arylgruppen gleich oder voneinander verschieden sind,
- R¹ eine Carboxygruppe umfasst, die protoniert oder geschützt ist oder auch nicht,
- zwei Gruppen von R², R³ und R⁴ H bedeuten und
- die eine Gruppe von R², R³ und R⁴, die kein H ist, eine Thioamidgruppe umfasst.

2. Calixarene nach Anspruch 1, worin R² und R⁴ H bedeuten und R³ eine Thioamidgruppe umfasst.

3. Calixarene nach Anspruch 1 oder 2, worin L zwischen allen Arylgruppen [-CH₂-] bedeutet.

4. Calixarene nach einem der Ansprüche 1 bis 3, worin R⁵ eine *tert*.-Butyl-Gruppe bedeutet.

5. Calixarene nach einem der Ansprüche 1 bis 4, worin die Carboxygruppe R¹ der allgemeinen Formel (A) entspricht:
(A) [- X - COOR¹⁰],
worin X eine C₁-, C₂- oder C₃-Kohlenstoffkette bedeutet, die ein Teil einer aliphatischen Kohlenwasserstoffgruppe, Arylgruppe oder Kohlenwasserstoffarylgruppe ist, wobei jede davon wahlweise durch eine oder mehrere Halogen-, Oxo- oder Nitrogruppen substituiert ist, und R¹⁰ H oder eine Schutzgruppe bedeutet, die ein Salz oder eine Estergruppe ist.

6. Calixarene nach Anspruch 5, worin R¹⁰ H bedeutet und die aliphatische Kohlenwasserstoffgruppe, Arylgruppe oder Kohlenwasserstoffarylgruppe mit der Formel (A) durch eine oder mehrere Gruppen substituiert ist, die eine Senkung des pKₐ der Carbonsäuregruppe in Bezug auf ein unsubstituiertes Molekül bewirken.

7. Calixarene nach Anspruch 5, worin R¹ die allgemeine Formel (B) besitzt:
(B) [-(C.R⁶.R⁷)ₙ-COOR¹⁰),
worin n 1, 2 oder 3 bedeutet und R⁶ und R⁷ H oder ein Halogenatom bedeuten und am jeweiligen Kohlenstoffatom gleich oder voneinander verschieden sind.

8. Calixarene nach Anspruch 5, worin R¹ die allgemeine Formel (C) besitzt: 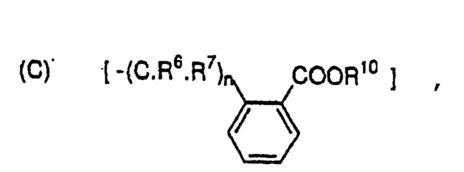 worin n 0 oder 1 bedeutet, R⁶ und R⁷ H oder ein Halogenatom bedeuten und am jeweiligen Kohlenstoffatom gleich oder voneinander verschieden sind und der Phenylring der Benzoesäuregruppe wahlweise durch eine oder mehrere Halogen-, Oxo- oder Nitrogruppen substituiert ist.

9. Calixarene nach Anspruch 8, worin R¹⁰ H bedeutet und der Phenylring der Benzoesäure mit der Formel (C) durch eine oder mehrere Gruppen substituiert ist, die eine Senkung des pKₐ der Carboxygruppe in Bezug auf ein unsubstituiertes Molekül bewirken.

10. Calixarene nach einem der Ansprüche 5 bis 9, worin n 1 bedeutet und R⁶ und R⁷ beide H bedeuten.

11. Calixarene nach einem der vorhergehenden Ansprüche, worin die Thioamidgruppe R², R³ oder R⁴ in Formel (I) die allgemeine Formel (D) besitzt: 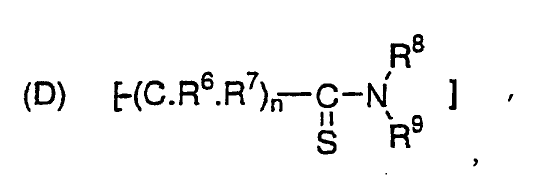 worin n 1, 2 oder 3 bedeutet, R⁶ und R⁷ H, ein Halogenatom oder eine aliphatische C₁- bis C₁₀-Kohlenwasserstoffgruppe bedeuten und am jeweiligen Kohlenstoffatom gleich oder voneinander verschieden sind und R⁸ und R⁹, die gleich oder voneinander verschieden sind, H oder eine aliphatische C₁- bis C₁₀-Kohlenwasserstoffgruppe bedeuten, die wahlweise durch eine oder mehrere Halogengruppen substituiert ist, wahlweise durch eine Ketogruppe unterbrochen ist oder wahlweise einen cycloaliphatischen Ring bedeutet, der von R⁸ und R⁹ zusammen gebildet wird, oder wahlweise an ein zweites Calixaren konjugiert ist.

12. Calixaren mit der Formel (II)

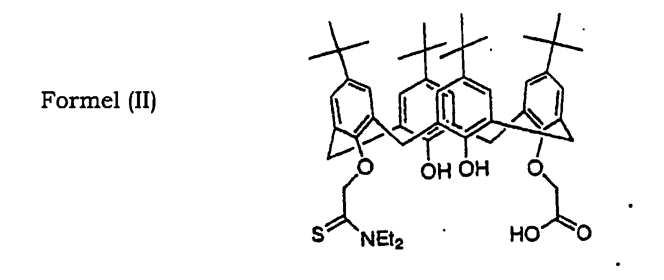

13. Calixarene mit der Formel (I) oder (II) nach Anspruch 1 oder 12, worin einige oder alle der Phenylgruppen des Calixarenrings weiterhin am Rand substituiert sind.

14. Calixaren-Dimer, das ein Calixaren nach Anspruch 11 enthält, worin eine der Gruppen R⁸ und R⁹ an ein zweites Calixaren konjugiert ist.

15. Dimer nach Anspruch 14, worin die R⁸- oder R⁹-Gruppe eines Calixarens an die R⁸- oder R⁹-Gruppe des anderen Calixarens konjugiert ist, wahlweise über eine Abstandshaltergruppe R¹¹, die eine aliphatische C₁- bis C₆-Kohlenwasserstoffgruppe, C₆- bis C₁₀-Arylgruppe, C₆- bis C₁₆-Kohlenwasserstoffarylgruppe bedeutet, wobei jede davon wahlweise durch eine oder mehrere Halogen- oder Oxogruppen substituiert oder durch eine oder mehrere Oxogruppen unterbrochen sein kann.

16. Verfahren zum Maskieren von Metallen, welches das Kontaktieren der Metalle mit einem Calixaren nach einem der vorhergehenden Ansprüche umfasst.

17. Verfahren nach Anspruch 16, das bei einem pH-Wert von zwischen 2 und 11 durchgeführt wird.

18. Verfahren nach Anspruch 16 oder 17, wobei der pH-Wert, bei welchem das Verfahren durchgeführt wird, gepuffert ist.

19. Verfahren nach Anspruch 18, worin der Puffer Citrat ist.

20. Verfahren nach einem der Ansprüche 16 bis 19, das folgende Stufen umfasst:
(i) Lösen des Calixarens in einem hydrophoben organischen Lösungsmittel,
(ü) Vermischen des organischen Lösungsmittels mit einer Metallionen enthaltenden wässrigen Phase,
(iii) Verrühren des organischen Lösungsmittels mit der wässrigen Phase und
(iv) Gewinnen des Metalls aus der organischen Phase.

21. Verfahren nach einem der Ansprüche 16 bis 20, worin das Metall aus Lanthaniden, U, Hg, Am, Pb, Sr, Bi, Cd, Ag und Y ausgewählt ist.

22. Verfahren nach einem der Ansprüche 16 bis 21, **dadurch gekennzeichnet ist, dass** das Calixaren an eine feste Phase gebunden ist.

23. Ionenselektive Polymermembran für einen elektrochemischen Sensor, die eine polymere Trägermatrix und ein Calixaren nach einem der Ansprüche 1 bis 15 umfasst.

24. Verfahren zur Herstellung eines Calixarens nach einem der Ansprüche 1 bis 15, das die aufeinanderfolgenden Stufen umfasst:
(i) Doppeltveresterung eines Calix[4]arens,
(ii) Entfernung der ersten Esterschutzgruppe, um eine erste Säuregruppe zu bilden,
(iii) Chlorierung der ersten Säuregruppe, um ein Säurechlorid zu bilden,
(iv) Substituierung der Chlorgruppe in dem Säurechlorid durch eine Diamin-Einheit, um eine Amidgruppe zu bilden, und
(i) Substituierung der Sauerstoffgruppe in der Amidgruppe durch eine Schwefelgruppe, um eine Thioamid-Einheit zu bilden.

25. Verfahren nach Anspruch 24, das eine anschließende Stufe umfasst, in der die zweite Esterschutzgruppe entfernt wird, um eine zweite Säuregruppe zu bilden.

## Revendications

1. Calixarènes de formule (I) : 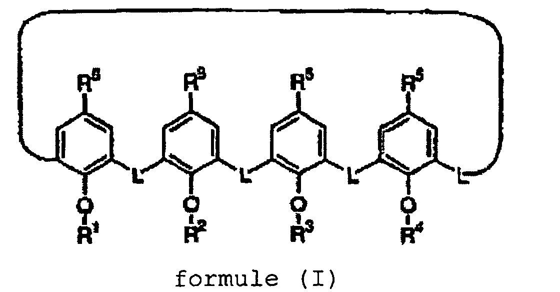 dans laquelle :
L est [-CH₂-] ou [-O-CH₂-O-] et est identique ou différent entre chaque groupe aryle ;
R⁵ est H, -NO₂, un halogène ou un groupe hydrocarbyle aliphatique en C₁ à C₁₀, un groupe aryle en C₆ à C₂₀, un groupe hydrocarbylaryle en C₆ à C₂₀, l'un quelconque de ces groupes étant éventuellement substitué par un ou plusieurs groupes halogéno ou oxo ou interrompu par un ou plusieurs groupes oxo, et R⁵ est identique ou différent sur chaque groupe aryle ;
R¹ comprend un groupe carboxy qui est ou non protoné ou protégé ;
deux groupes parmi R², R³ et R⁴ sont H ; et
le groupe parmi R², R³ et R⁴ qui n'est pas H comprend un groupe thioamide.

2. Calixarènes selon la revendication 1, dans lesquels R² et R⁴ sont H et R³ comprend un groupe thioamide.

3. Calixarènes selon la revendication 1 ou la revendication 2, dans lesquels L est [-CH₂-] entre chacun des groupes aryle.

4. Calixarènes selon l'une quelconque des revendications 1 à 3, dans lesquels R⁵ est un groupe tert-butyle.

5. Calixarènes selon l'une quelconque des revendications 1 à 4, dans lesquels le groupe carboxy R¹ est conforme à la formule générale (A) :
(A) [-X-COOR¹⁰]
dans laquelle X est une chaîne carbonée en C₁, C₂ ou C₃ qui constitue une partie d'un groupe hydrocarbyle aliphatique, d'un groupe aryle ou d'un groupe hydrocarbylaryle, l'un quelconque de ces groupes étant éventuellement substitué par un ou plusieurs groupes halogéno, oxo ou nitro ; et R¹⁰ est H ou un groupe protecteur qui est un sel ou un groupe ester.

6. Calixarènes selon la revendication 5, dans lesquels R¹⁰ est H et le groupe hydrocarbyle aliphatique, le groupe aryle ou le groupe hydrocarbylaryle dans la formule (A) est substitué par un ou plusieurs groupes qui provoquent une réduction du pKa du groupe acide carboxylique par rapport à une molécule non substituée.

7. Calixarènes selon la revendication 5, dans lesquels R¹ répond à la formule générale (B) :
(B) [-( C.R⁶.R⁷)ₙ-COOR¹⁰]
dans laquelle n vaut 1, 2 ou 3 et R⁶ et R⁷ sont H ou des halogènes et sont identiques ou différents sur chaque carbone.

8. Calixarènes selon la revendication 5, dans lesquels R¹ répond à la formule générale (C) : 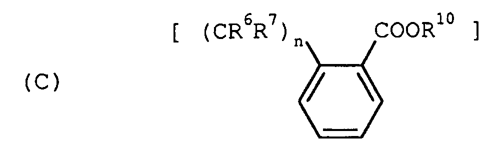 dans laquelle n vaut 0 ou 1 et R⁶ et R⁷ sont H ou des halogènes et sont identiques ou différents sur chaque carbone, et dans laquelle le cycle phényle du groupe acide benzoïque est éventuellement substitué par un ou plusieurs groupes halogéno, oxo ou nitro.

9. Calixarènes selon la revendication 8, dans lesquels R¹⁰ est H et le cycle phényle de l'acide benzoïque de formule (C) est substitué par un ou plusieurs groupes qui provoquent une réduction du pKa du groupe carboxy par rapport à une molécule non substituée.

10. Calixarènes selon l'une quelconque des revendications 5 à 9, dans lesquels n vaut 1 et R⁶ et R⁷ sont tous deux H.

11. Calixarènes selon l'une quelconque des revendications précédentes, dans lesquels le groupe thioamide R², R³ ou R⁴ dans la formule (I) répond à la formule générale (D) : 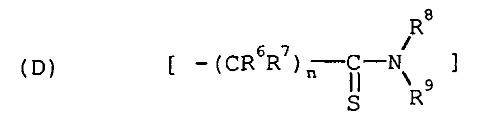 dans laquelle n vaut 1, 2 ou 3, et R⁶ et R⁷ sont H, des halogènes ou des groupes hydrocarbyle aliphatiques en C₁ à C₁₀, et sont identiques ou différents sur chaque carbone, et dans laquelle R⁸ et R⁹, qui sont identiques ou différents, sont H ou des groupes hydrocarbyle aliphatique en C₁ à C₁₀ qui sont éventuellement substitués par un ou plusieurs groupes halogéno, ou sont éventuellement interrompus par un groupe céto ou sont éventuellement des cycles aliphatiques formés par R⁸ et R⁹ ensemble, ou sont éventuellement conjugués à un deuxième calixarène.

12. Calixarène de formule (II) :

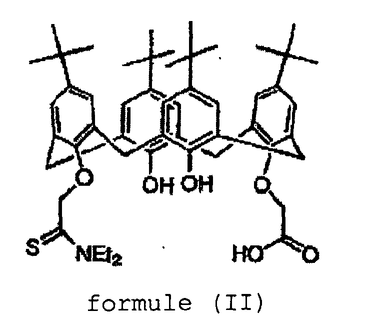

13. Calixarènes de formule (I) ou (II) selon la revendication 1 ou 12, dans lesquels tout ou partie des groupes phényle du cycle calixarène sont en outre substitués sur leur périphérie.

14. Dimère de calixarène comprenant un calixarène selon la revendication 11 dans lequel l'un des groupes R⁸ et R⁹ est conjugué à un deuxième calixarène.

15. Dimère selon la revendication 14, dans lequel le groupe R⁸ ou R⁹ d'un calixarène est conjugué au groupe R⁸ ou R⁹ de l'autre calixarène, éventuellement par l'intermédiaire d'un groupe d'espacement R¹¹, le groupe d'espacement facultatif R¹¹ étant un groupe hydrocarbyle aliphatique en C₁ à C₆, un groupe aryle en C₆ à C₁₀, un groupe hydrocarbylaryle en C₆ à C₁₆, l'un quelconque de ces groupes pouvant éventuellement être substitué par un ou plusieurs groupes halogéno ou oxo ou interrompu par un ou plusieurs groupes oxo.

16. Procédé pour séquestrer des métaux, comprenant la mise en contact des métaux avec un calixarène selon l'une quelconque des revendications précédentes.

17. Procédé selon la revendication 16, dans lequel le procédé est mis en oeuvre à un pH compris entre 2 et 11.

18. Procédé selon la revendication 16 ou 17, dans lequel le pH auquel le procédé est mis en oeuvre est tamponné.

19. Procédé selon la revendication 18, dans lequel le tampon utilisé est du citrate.

20. Procédé selon l'une quelconque des revendications 16 à 19, comprenant les étapes suivantes :
(i) dissolution du calixarène dans un solvant organique hydrophobe ;
(ii) mélange du solvant organique avec une phase aqueuse contenant des ions métalliques ;
(iii) agitation du solvant organique et de la phase aqueuse ensemble ;
(iv) récupération du métal à partir de la phase organique.

21. Procédé selon l'une quelconque des revendications 16 à 20, dans lequel le métal est choisi parmi un lanthanide, U, Hg, Am, Pb, Sr, Bi, Cd, Ag et Y.

22. Procédé selon l'une quelconque des revendications 16 à 21, **caractérisé en outre en ce que** le calixarène est lié en phase solide.

23. Membrane polymère sélective d'ions pour un détecteur électrochimique comprenant une matrice polymère de support et un calixarène selon l'une quelconque des revendications 1 à 15.

24. Procédé pour préparer un calixarène selon l'une quelconque des revendications 1 à 15, comprenant les étapes successives suivantes :
(i) bis-estérification d'un calix[4]arène ;
(ii) déprotection d'un premier groupe ester pour former un premier groupe acide ;
(iii)chloration du premier groupe acide pour former un chlorure d'acyle ;
(iv) remplacement du groupe chlore dans ledit chlorure d'acyle par un fragment diamine pour former un groupe amide ; et
(v) remplacement du groupe oxygène dans le groupe amide par un groupe soufre pour former un fragment thioamide.

25. Procédé selon la revendication 24, dans lequel le procédé comprend une étape ultérieure de déprotection du deuxième groupe ester pour former un deuxième groupe acide.
